# EUROPÄISCHE PATENTSCHRIFT

(11) **EP 1 658 041 B1**
(45) Veröffentlichungstag und Bekanntmachung des Hinweises auf die Patenterteilung: **17.09.2008**
(21) Anmeldenummer: 04764512.2
(22) Anmeldetag: 26.08.2004
(51) Int. Cl.: A61K 8/18, A61K 33/38

(54) **KÖRPERPFLEGEMITTEL MIT PORÖSEN SILBERPARTIKELN**
BODY CARE PRODUCT CONTAINING POROUS SILVER PARTICLES
PRODUIT DE SOIN CORPOREL CONTENANT DES PARTICULES POREUSES D'ARGENT

(30) Priorität: 29.08.2003 DE 10340277
(43) Veröffentlichungstag der Anmeldung: 24.05.2006
(73) Patentinhaber: Bio-Gate AG, 90411 Nürnberg (DE)
(72) Erfinder: BECHERT, Thorsten, 91301 Forchheim (DE); WAGENER, Michael, 28355 Bremen (DE); STEINRÜCKE, Peter, 91052 Erlangen (DE)
(74) Vertreter: Ehnis, Tobias
(86) Internationale Anmeldenummer: PCT/EP2004/009536
(87) Internationale Veröffentlichungsnummer: WO 2005/023213

(56) Entgegenhaltungen:
- WO-A-00/78281
- WO-A-02/17984
- US-A- 4 457 460
- US-A- 4 828 832
- US-A- 4 906 466
- US-A- 5 290 544
- US-A- 5 595 750

## Beschreibung

Die Erfindung betrifft ein Körperpflegemittel sowie eine Verwendung zur Herstellung eines Medikaments zur Behandlung einer Entzündung und/oder Infektion.

Aus der WO 02/17984 A1 ist ein antimikrobielles Material zum Implantieren in Knochen oder zum Beschichten oder Herstellen eines Implantats oder einer implantierbaren medizinischen Vorrichtung bekannt. Dabei sind aus einem antimikrobiellen Metall gebildete Partikel in einem im ausgehärteten Zustand eine Matrix bildenden Matrixmaterial fein verteilt. Das Metall kann aus einem oder mehreren der folgenden Bestandteile gebildet sein: Ag, Au, Pt, Pd, Ir, Sn, Cu, Sb, Zn. Das Metall ist aus Aggregaten von Primärpartikeln mit einer mittleren Korngröße zwischen 10 und 100 nm gebildet. Die Aggregate können eine mittlere Korngröße von 1 bis 20 µm, vorzugsweise 10 bis 20 µm, und eine Porosität von 70 bis 95% aufweisen.

Aus US 5,595,750 ist eine antimikrobielle Mischung mit Partikeln aus einem Kernmaterial bekannt, welches aus einem Metalloxid, einem Sulfat, einem Sulfid, einem Zeolith, Glimmer, Talg, Kaolin, Silika oder Mullit gebildet ist.

Die US 4,828,832 offenbart eine zur Behandlung von Hautverletzungen einzusetzende Zusammensetzung von fein verteilten Silberpartikeln in einem aus gebranntem Ton bestehenden Träger. Die Silberpartikel können eine Größe von 1 bis 10 µm aufweisen.

US 4,906,466 offenbart eine antimikrobielle Zusammensetzung zur Anwendung auf oder Imprägnierung in medizinischen oder anderen Geräten, zur Inkooperation in eine Beschichtung oder eine Imprägnierungsformulierung für solche Geräte oder für oberflächliche Anwendungen, bei welchen der Erhalt von sterilen oder kontaminationsresistenten Bedingungen erforderlich ist. Die Zusammensetzung enthält eine Silberverbindung, welche auf einem physiologisch inerten Träger abgeschieden ist. Bei der Silberverbindung kann es sich um Silberchlorid handeln. Der Träger besteht aus einem Partikel aus Metalloxid, Kalziumhydroxylapatit oder Bariumsulfat. Die Partikelgröße des Trägermaterials kann 1 bis 15 µm betragen.

Aus der WO 00/78281 A1 ist ein antimikrobielles Körperpflegemittel bekannt, welches in einem menschliche oder tierische Haut und/oder Mukosa kontaktierenden Teil eine organische Matrix aufweist. Diese Matrix enthält homogen dispergierte Partikel von metallischem Silber. Die Partikel haben dabei eine Größe zwischen 1 und 50 nm. Bei Partikeln dieser Größe handelt es sich um so genannte Nanopartikel. Diese Partikel sind in einer Menge enthalten, welche auf der Oberfläche des die Haut und/oder Mukosa kontaktierenden Teils eine antimikrobiell wirksame aber weniger als zytotoxische Konzentration bereitstellt. Bei dem Körperpflegemittel kann es sich beispielsweise um eine Salbe oder eine Creme handeln.

Aus Brumfiel, G. Nature (2003), Bd. 424, Seiten 246 bis 248 ist es bekannt, dass Nanopartikel von Tieren aufgenommen werden können. Nanopartikel können beispielsweise aus der Lunge in den Blutstrom gelangen. Es ist bisher nicht klar, welche Auswirkungen Nanopartikel auf die menschliche Gesundheit haben, wenn sie in den Körper eingedrungen sind. Die Auswirkungen der in dem Körperpflegemittel gemäß der WO 00/78281 A1 enthaltenen aus Silber bestehenden Nanopartikel auf die menschliche Gesundheit sind daher ebenfalls unklar.

Aus der DE 693 21 139 T2 ist eine antimikrobielle Zusammensetzung aus anorganischen Teilchen, die mit metallischem Silber beschichtet sein können, bekannt. Die Teilchen können in einem Polymer eingearbeitet sein. Sie weisen einen Durchmesser von 0,01 bis 100 µm auf, d. h. sie können auch in Form von Nanopartikeln vorliegen. Die der DE 693 21 139 T2 zu Grunde liegende Aufgabe besteht darin, antimikrobielle Teilchen bereitzustellen, welche sich gut in eine Polymermatrix einbauen lassen, wobei die Wechselwirkung mit dem Polymer minimiert wird. Die Partikel weisen über dem Silber eine Schutzschicht geringer Porosität auf und sind in dem Polymer gut dispergierbar. Die Schutzschicht soll verhindern, dass das Metall mit seiner Umgebung zu intensiv in Verbindung tritt.

Aus der DE 38 86 193 T2 ist ein Titan-Glimmer-Verbundmaterial bekannt, welches als Pigment in Kosmetika eingesetzt werden kann und das eine Beschichtung aus metallischem pulverförmigen Silber aufweist. Die der DE 38 86 193 T2 zu Grunde liegende Aufgabe besteht in der Bereitstellung eines Materials, das sich zur Verwendung als Färbemittel oder Pigment eignet.

Aus der WO 00/78282 A1 ist eine Silikonkautschuk-Verbindung bekannt, welche 1 bis 50 nm große metallische Silberpartikel enthält. Die Silberpartikel sind in einer Menge enthalten, welche auf der Oberfläche der genannten Verbindung eine antimikrobiell wirksame aber weniger als zytotoxische Silberkonzentration bereitstellt.

Aus der JP 61257908 A ist eine Make-up Zusammensetzung bekannt, die ein Pulver enthält, welches mit einem Metallpulver, z. B. aus Silber, beschichtet ist. Offensichtlich dient hier die Silberbeschichtung ausschließlich der Bereitstellung optischer Eigenschaften.

Aufgabe der vorliegenden Erfindung ist es, ein antimikrobiell wirksames Körperpflegemittel bereitzustellen, welches die mit Nanopartikeln verbundenen Nachteile des aus der WO 00/78281 A1 bekannten Körperpflegemittels nicht aufweist. Weiterhin soll eine Verwendung zur Herstellung eines Medikaments zur Behandlung einer Entzündung bei einem Säugetier oder Menschen angegeben werden.

Die Aufgabe der vorliegenden Erfindung wird durch die Merkmale der Patentansprüche 1 und 16 gelöst. Zweckmäßige Ausgestaltungen ergeben sich aus den Merkmalen der Ansprüche 2 bis 15 und 17 bis 31.

Erfindungsgemäß ist ein Körperpflegemittel vorgesehen, welches metallisches Silber enthaltende aus Metall gebildete poröse Partikel mit einem mittleren Durchmesser zwischen 1 und 100 µm enthält.

Körperpflegemittel sind Produkte, welche mit der menschlichen oder tierischen Haut und/oder Mukosa in Kontakt gebracht werden, um eine reinigende, schützende, therapeutische, heilende, pflegende, kosmetische oder lindernde Wirkung zu erzielen. Beispielsweise sind das Produkte, die üblicherweise Oberflächen aufweisen, welche die Haut kontaktieren und aus einem natürlichen oder synthetischen Polymermaterial bestehen. Das können z. B. absorbierende Einwegartikel, wie Damenhygieneartikel, insbesondere Monatsbinden, Slipeinlagen oder Tampons, Inkontinenzeinlagen, Windeln, Trainingskinderhöschen, medizinische Binden, Pflaster, Vliese, Textilien, Zellstoffe, Zahnbürsten oder Schnuller sein. Die Körperpflegemittel können aus einem Naturstoff, wie Wolle, Viskose, Zellulose und davon abgeleiteten Derivaten oder Naturkautschuk hergestellt sein oder diese Naturstoffe enthalten. Sie können auch aus Kunststoffen hergestellt sein oder Kunststoffe enthalten, welche die metallisches Silber enthaltenden porösen Partikel enthalten. Die Kunststoffe können z.B. sein: Polyethylene und daraus abgeleitete Copolymere, Polypropylene und daraus hergestellte Polyblends, Polybutene, Polystyrole in Homo- und Copolymerisaten, Acryl-Butadien-Styrol-Terpolymerisat (ABS), Synthesekautschuks, Hart- und Weich-PVC, Polytetrafluorethen (PTFE), Polychlortrifluorethylen (PCTFE) und andere Fluorpolymere, Polyvinylether, Polyvinylacetate, Polyvinylpropionate, Polyvinylalkohole, Copolymere des Vinylalkohols, Polyvinylacetale, Polyethylenglykole, Acrylpolymerisate, Polymetacrylsäuremethylester, Polyacrylnitril, Polycyanoacrylate, Polymere auf Polymethacrylimidbasis, Polyacrylimide, Polyvinylamine, Polyamide einschließlich Polyphenylenisophtalamid, Poly (p-phenylenterephtalamid), lineare Polyurethane und Polyester einschließlich Polyethylenterephthalat (PET), Polybutylen- terephthalat (PBT) und Polytetramethylenterephthalat (PTMT), Polycarbonate und daraus abgeleitete Polymere, Polyoxymethylene (POM), Polyether, Polyetheretherketone, Polyetherblockamide, Kondensationsharze, wie Phenolplaste und Aminoplaste, vernetzte Polyester einschliesslich Polyesterharze, Epoxyharze, vernetzte Polyurethane, Reaktionsharze auf Methylmethacrylat-Basis, Polysiloxane und andere Polymere mit anorganischer Hauptkette.

Die Körperpflegemittel können auch, insbesondere medizinisch wirksame, Präparate sein, wie Emulsionen, Lotionen, Gele, Cremes, Salben, Heilsalben, Puder, Kosmetika, Hautschutzcremes oder -salben, Desinfektionsmittel oder antiinflammatorische Heilmittel, Suspensionen, Seifen, synthetische Tenside, Badezusätze, Peelingpräparate, Gesichtswässer, Zahnpflegemittel, Zahncremes, Mundwässer, Zahnreinigungskaugummis, Prothesenhaftmittel, Haarshampoos, Sonnenschutzmittel, etc.. Diese Produkte enthalten häufig entweder ein Polymer oder einen organischen Bestandteil in einem Träger, welcher ein gutes Substrat für eine Vielzahl von Mikroorganismen sein kann. Ein Wachstum dieser Mikroorganismen in diesen Substraten kann hygienische oder medizinische Probleme verursachen.

Die Partikel können in dem Körperpflegemittel in einer Menge enthalten sein, die an einer Stelle des Kontakts des Körperpflegemittels mit der Haut und/oder Mukosa eine antimikrobiell wirksame aber weniger als zytotoxische Konzentration an Silber-Ionen ermöglicht.

Die in dem erfindungsgemäßen Körperpflegemittel enthaltenen aus Metall bestehenden Partikel weisen auf Grund ihrer Größe von 1 bis 100 µm nicht die potenziellen Risiken von Nanopartikeln auf. Die Partikel können beim bestimmungsgemäßen Gebrauch des Körperpflegemittels nicht durch tiefere Hautschichten hindurch ins Gewebe oder in Blutgefäße eindringen und weiterhin können sie auch nicht die Blut-Hirn-Schranke überwinden. Dadurch ist der antimikrobielle Effekt allein auf die Hautoberfläche beschränkt. Die Auslösung von Allergien und unerwünschten toxischen Effekten wird dadurch vermieden. Es wurde aber festgestellt, dass die Silber-Ionen, die von den Partikeln auf Grund ihrer Porosität freigesetzt werden können, ausreichen, um ein antimikrobiell und gegebenenfalls antiinflammatorisch wirksames Körperpflegemittel bereitstellen zu können. Eine antiinflammatorische Wirkung kann erzielt werden, wenn die Partikel in einer höheren Konzentration in dem Körperpflegemittel enthalten sind als zur Erreichung einer bloß antimikrobiellen Wirkung erforderlich ist. Die Silber-Ionen wirken vor allem auf der Oberfläche der das Körperpflegemittel kontaktierenden Haut- bzw. Schleimhaut und haben keinen negativen Einfluss auf darunter liegendes Gewebe. Die Partikel sind dadurch und wegen ihrer ein Eindringen in die Haut verhindernden Größe sehr viel hautverträglicher als Nanopartikel. Die Partikel sind weniger zellschädigend und biokompatibler als metallisches Silber enthaltende Nanopartikel. Das erfindungsgemäße Körperpflegemittel ist dadurch insbesondere für Patienten geeignet, die dauerhaft eine gesteigerte Sorgfalt auf Körperpflege und Körperhygiene verwenden müssen. Das können z.B. Personen sein, die ein geschwächtes Immunsystem und/oder ein erhöhtes Risiko haben, an Hautinfektionen zu erkranken, wie beispielsweise Diabetiker. Da festgestellt worden ist, dass das erfindungsgemäße Körperpflegemittel häufig die zusätzliche Anwendung von Antibiotika überflüssig macht, kann dadurch auch der Entstehung von Antibiotika-Resistenzen vorgebeugt werden.

Das erfindungsgemäße Körperpflegemittel wirkt antimikrobiell und gegebenenfalls gleichzeitig antiinflammatorisch. Darüber hinaus benötigt es wegen der antimikrobiellen Wirkung des metallischen Silbers neben den Partikeln keine Konservierungsstoffe. Es kann als, insbesondere medizinische/s, Heil- oder Pflegesalbe, -creme oder -gel ausgebildet sein. Ein solches Präparat kann wegen der entzündungshemmenden Wirkung alternativ zu Kortikoid enthaltenden Präparaten medizinisch angewandt werden. Als Handsalbe, -creme oder -gel schützt die antimikrobielle Wirkung auch vor dem Übertragen von Krankheitserregern, z. B. durch Händeschütteln, und verhindert bei kleinen Wunden an den Händen das Eindringen von Keimen. Dadurch, dass auf Konservierungsstoffe verzichtet werden kann, treten darüber hinaus weniger, insbesondere allergische, Unverträglichkeitsreaktionen auf.

Die Partikel weisen bevorzugt eine mittlere innere Porosität von mindestens 65%, insbesondere zwischen 65 und 95% auf. Unter innerer Porosität wird der prozentuale Anteil des Volumens des Partikels verstanden, der nicht von Metall ausgefüllt ist. Die mittlere innere Porosität der Partikel kann nach folgendem Verfahren bestimmt werden:
1. Einbetten der Partikel in einen Kunststoff,
2. Herstellung von Ultradünnschnitten der eingebetteten Partikel,
3. Anfertigen von Transmissions-Elektronenmikroskop(TEM)-Aufnahmen der Partikel,
4. Bestimmung des prozentualen Anteils der nicht von Metall ausgefüllten Fläche jeweils innerhalb eines Partikels im Verhältnis zur Gesamtfläche dieses Partikels in einer Mehrzahl der TEM-Aufnahmen und
5. Berechnen des Mittelwerts einer Mehrzahl so bestimmter prozentualer Anteile.

Der Schritt Nr. 4 kann dabei durch eine computergestützte Bildauswertung der TEM-Aufnahmen erfolgen. Neben der inneren Porosität kann auch die Gesamtporosität der Partikel bestimmt werden. Dazu wird zunächst die Klopfdichte eines Pulvers der Partikel bestimmt. Die Klopfdichte ist die Masse einer volumeneinheit eines durch Klopfen möglichst dicht gelagerten Pulvers. Die Klopfdichte kann nach DIN ISO 3953 bestimmt werden. Der dabei ermittelte Wert wird als prozentualer Anteil an der Dichte des die Partikel bildenden Metalls, hier Silber mit einer Dichte von 10,49 g/cm³, berechnet und von 100% subtrahiert. Der so berechnete Wert stellt die Gesamtporosität der Partikel dar. Er kann für die in dem erfindungsgemäßen Körperpflegemittel enthaltenen Partikel zwischen 85 und 95%, insbesondere zwischen 90 und 95%, vorzugsweise zwischen 93 und 95% liegen.

Besonders vorteilhaft ist es, wenn die Partikel eine mittlere innere Porosität zwischen 65 und 90%, insbesondere zwischen 70 und 85%, vorzugsweise zwischen 75 und 85%, oder zwischen 85 und 95%, vorzugsweise zwischen 90 und 95%, aufweisen. Durch die Wahl der Porosität lässt sich die Menge der Silber-Ionen festlegen, die von einem Partikel in eine bestimmten Zeiteinheit freigesetzt werden. Wird eine große Porosität gewählt, werden dadurch viele Silber-Ionen freigesetzt, so dass insgesamt mit einer geringeren Silbermenge in dem Körperpflegemittel die antimikrobielle und antiinflammatorische Wirkung erreicht wird. Auf der anderen Seite wird durch eine Erhöhung der Porosität und gleichzeitiger Verringerung der Silbermenge die Gesamtdauer der Silber-Ionen-Freisetzung verringert. Je nach Anwendung ist daher eine Porosität zwischen 70 und 85% oder zwischen 85 und 95% vorteilhaft.

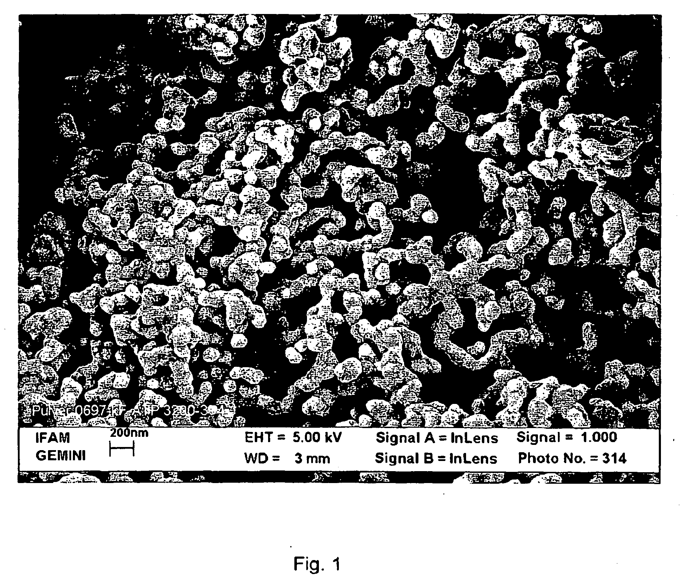

Vorzugsweise liegen die Partikel als Agglomerate metallischer Primärpartikel vor. Die Agglomerate können aus Primärpartikeln mit einem mittleren Durchmesser zwischen 10 und 200 nm, vorzugsweise zwischen 15 und 80 nm, gebildet sein. Primärpartikel dieser Größe erlauben eine ausreichende Freisetzung von Silber-Ionen und sind gut herzustellen. Der mittlere Abstand zwischen den jeweils äußersten Primärpartikeln an der Oberfläche der Agglomerate liegt bevorzugt im Bereich von 20 bis 200 nm, vorzugsweise 100 bis 200 nm. Die Primärpartikel lassen sich auf Grund ihrer äußeren Form und Größe elektronenmikroskopisch identifizieren. Sie sind bspw. in Fig. 1 als kugelige Gebilde zu erkennen. Die Primärpartikel sind miteinander über Sinterhälse verbunden.

Die porösen Partikel weisen vorzugsweise eine schwammartige Struktur auf. Durch die dadurch bereitgestellte große Oberfläche können Silber-Ionen in ausreichender Menge freigesetzt werden, um antimikrobiell und gegebenenfalls antiinflammatorisch wirksam zu sein.

Bevorzugt weisen die Partikel einen mittleren Außendurchmesser von 2 bis 20 µm, vorzugsweise 2 bis 5 µm, auf. Die spezifische Oberfläche der Partikel kann zwischen 2 und 10 m²/g, insbesondere zwischen 3 und 6 m²/g, vorzugsweise zwischen 3,5 und 4,5 m²/g, liegen. Die spezifische Oberfläche kann z.B. mittels N₂ - Adsorption volumetrisch nach der BET-Methode bestimmt werden. Die BET-Methode ist eine nach Brunauer, Emmett und Teller benannte Methode zur Bestimmung der Oberfläche und gegebenenfalls auch der Poren-Größenverteilung von festen Körpern (z.B. Pulvern), die davon ausgeht, dass Gase, Dämpfe etc. auf festen Körpern unter Freisetzung einer messbaren Adsorptionswärme zunächst in einer monomolekularen Schicht adsorbiert werden. Beispielsweise kann das Volumen an Stickstoff-Gas, das bei -196°C in Abhängigkeit vom angewandten Druck auf dem Adsorptionsmittel adsorbiert wird, gemessen werden.

Vorzugsweise bestehen die Partikel mindestens zu 99% w/w (Gewichtsprozent), vorzugsweise zu 99,9% w/w, aus metallischem Silber. Bei einem solch hohen Silbergehalt kommt ein zytotoxischer Effekt anderer Metall-Ionen, insbesondere von KupferIonen, nicht zum Tragen. Die den Metallgehalt angebenden Prozentangaben beziehen sich hier und im Folgenden wenn nicht anders angegeben auf den Gewichtsanteil der angegebenen Metalle am Gesamtgewicht der Partikel. Es sind Angaben von Gewichtsprozenten (% w/w). Besonders vorteilhaft ist es, wenn die Partikel Verunreinigungen von weniger als 5 ppm an Kalium, Natrium oder Chlor aufweisen. Größere Verunreinigungen des Silbers können unerwünschte Nebenwirkungen auslösen.

Besonders vorteilhaft ist es, wenn die Partikel bis zu 0,5% w/w metallisches Zink und/oder bis zu 0,5% w/w metallisches Kupfer enthalten. Beide Stoffe wirken ebenfalls antimikrobiell und unterstützen sich zusammen mit Silber gegenseitig in der Wirkung. Das liegt u.a. daran, dass sie in ihrer antimikrobiellen Wirkung eine unterschiedliche Spezifität für Mikroorganismen aufweisen. Darüber hinaus weist Zink in Kombination mit Silber und gegebenenfalls Kupfer eine besonders gute wundheilende und entzündungshemmende Wirkung auf. Ursache dafür könnte sein, dass durch das Silber und das gegebenenfalls enthaltene Kupfer das Wachstum von die Wundheilung störenden Mikroorganismen unterbunden wird, deren Wachstum durch Zink-Ionen alleine nicht gehemmt wird. Das Kupfer erleichtert darüber hinaus die Herstellung einer Legierung aus Zink und Silber. Insgesamt hat das neben Silber Zink und/oder Kupfer enthaltende Körperpflegemittel eine bessere wundheilende und entzündungshemmende Wirkung, als ein Körperpflegemittel, das jeweils nur eines der Metalle enthält. Vorzugsweise sind die Partikel aus einer Silber-Zink-Legierung oder einer Silber-Zink-Kupfer-Legierung gebildet.

Neben den Partikeln sind in dem Körperpflegemittel vorzugsweise keine Konservierungsstoffe enthalten. Es ist festgestellt worden, dass die Metall-Ionen eine konservierende Wirkung aufweisen. Daher kann auf Konservierungsstoffe verzichtet werden. Unerwünschte, insbesondere allergische von einem üblichen Konservierungsstoff, wie z. B. Formaldehyd, ausgelöste Reaktionen können dadurch vermieden werden.

Die Partikel können in einem Trägermaterial enthalten sein, welches aus einem Silikonöl, einem Mineralöl, Glyzerin oder einem üblichen aus der Pharmakologie bekannten Salbenbestandteil besteht. Zur Herstellung eines erfindungsgemäßen Körperpflegemittels können die Agglomerate durch thermisches Verdampfen des die Agglomerate bildenden Metalls und anschließendem Abscheiden des Metalldampfs auf einem Metallfilter hergestellt werden. Die Agglomerate können in ein Trägermaterial aufgenommen werden, das in das Körperpflegemittel eingebracht wird. Das Trägermaterial kann beispielsweise ein Silikonöl, ein Mineralöl, Glyzerin oder ein üblicher aus der Pharmakologie bekannter Salbenbestandteil sein.

Darüber hinaus betrifft die Erfindung die Verwendung von metallisches Silber enthaltenden aus Metall gebildeten porösen Partikeln mit einem mittleren Durchmesser zwischen 1 und 100 µm zur Herstellung eines Medikaments zur Behandlung einer Entzündung und/oder Infektion bei einem Säugetier oder Menschen. Übliche Medikamente zur Behandlung einer Entzündung bei einem Säugetier oder Menschen weisen oft eine Kombination von antiinflammatorischen und antimikrobiellen Wirkstoffen auf. Der antimikrobielle Wirkstoff soll eine Infektion, insbesondere mit Staphylococcus aureus, verhindern oder bekämpfen. Üblicherweise handelt es sich bei dem antimikrobiellen Wirkstoff um ein Antibiotikum. Alternativ kann das Antibiotikum auch systemisch verabreicht werden, während der antiinflammatorische Wirkstoff lokal, z.B. topisch, verabreicht wird. Wegen der, insbesondere bei langfristiger Anwendung, bestehenden Gefahr der Entstehung von Antibiotika-Resistenzen sollte der Einsatz von Antibiotika jedoch auf ein Mindestmaß reduziert werden. Als antiinflammatorischer Wirkstoff wurde bisher z. B. ein Kortikoid wie Kortison verwendet, das jedoch eine große Zahl von Nebenwirkungen aufweist. Der wesentliche Vorteil des erfindungsgemäß hergestellten Medikaments besteht darin, dass die Partikel sowohl eine antiinflammatorische als auch eine antimikrobielle Wirkung aufweisen. Der Einsatz von Antibiotika kann reduziert und die Nebenwirkungen der Kortikoide oder anderer antiinflammatorischer Wirkstoffe können vermieden werden.

Die Behandlung erfolgt vorzugsweise topisch, d.h. beispielsweise durch Auftragen auf die Haut oder eine Wunde. Das Medikament kann eine Salbe, eine Creme oder ein Gel sein. Weitere vorteilhafte Ausgestaltungen der Verwendung ergeben sich aus den vorangehenden, das erfindungsgemäße Körperpflegemittel betreffenden Ausführungen.

Nachfolgend wird die Erfindung anhand von Ausführungsbeispielen näher erläutert. Es zeigen:
- Fig. 1: eine rasterelektronenmikroskopische Aufnahme eines Silber-Agglomerats und
- Fig. 2: eine Matrix grafischer Darstellungen des zeitlichen Verlaufs des in Form von optischer Dichte (OD) eines Mediums gemessenen Wachstums von Bakterien in Kontakt mit verschiedenen cremeförmigen Körperpflegemitteln.

Fig. 1 zeigt eine rasterelektronenmikroskopische Aufnahme eines Silber-Agglomerats. Das Silber-Agglomerat besteht hier im Wesentlichen aus kugeligen Primärpartikeln mit einer mittleren Korngröße von etwa 60 nm. Die Primärpartikel sind im wesentlichen über Sinterhälse miteinander verbunden. Sie bilden ein hochporöses Gerüst. Das hier gezeigte Silber-Agglomerat hat eine Größe von etwa 10 µm.

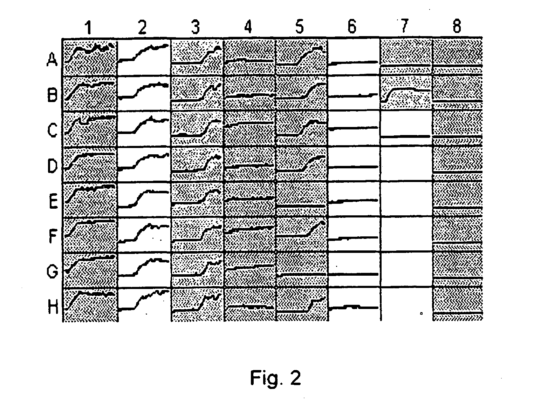

Die in Fig. 2 gezeigten Ergebnisse sind nach dem aus der DE 197 51 581 A1 bekannten Verfahren ermittelt worden. Dieses Verfahren ist ferner beschrieben in Bechert, Thorsten et al., Nature Medicine (2000), Bd. 6, Nr. 8, Seiten 1053 bis 1056. Der Offenbarungsgehalt der beiden vorgenannten Dokumente wird hier einbezogen. Die zu testenden erfindungsgemäßen Körperpflegemittel wurden in Form von Cremes hergestellt, jeweils auf einem Werkstoff als Träger aufgetragen und in dem Test wie beschrieben eingesetzt. Im einzelnen wurde der Test wie folgt durchgeführt:

Es werden zunächst verschiedene Cremeproben hergestellt. Auf jeden Träger wird eine Menge von 11 mg der jeweiligen Creme aufgetragen. Anschließend werden in jede Vertiefung einer Mikrotiterplatte 200 µl einer Staphylococcus epidermidis enthaltenden Lösung gefüllt. Die Träger mit den Cremeproben werden jeweils in einer der Vertiefungen bei 37°C für eine Stunde inkubiert. Die Träger werden dann entnommen und dreimal mit physiologischem Puffer gewaschen. Anschließend werden die Träger jeweils in eine Vertiefung einer Mikrotiterplatte gelegt, welche mit 200 µl eines Minimalmediums gefüllt ist. Die Träger werden für 24 Stunden bei 37°C inkubiert. Anschließend werden die Träger entnommen und verworfen. Zu jeder Vertiefung der Mikrotiterplatte werden 50 µl eines Vollmediums (Trypcase-soja, bioMerieux,Nr. 69280, Marcy l'Etoile, Frankreich) zugegeben. Anschließend wird die Trübung der Lösung im Abstand von 30 Minuten über einen Zeitraum von 48 Stunden gemessen. Die Lösung wird dabei auf einer Temperatur von 37°C gehalten. Die Trübungsmessung erfolgt mit Licht einer Wellenlänge von 578 nm mittels eines geeigneten Lesegeräts. Eine Trübung zeigt an, dass Bakterien von der Oberfläche des Trägers in die Umgebung abgegeben worden sind.

Zur Herstellung der Cremeproben wurde als Basiscreme "Cremaba Plus HT" der Firma Spinnrad^{®}, Certus Handels GmbH, 22848 Norderstedt, Deutschland, verwendet. Dabei handelt es sich um eine Emulsionsgrundlage mit folgenden Inhaltsstoffen: Aqua, Caprylic/Capric Triglyceride, Pentylene Glycol, Hydrogenated Lecithin, Butyrospermum Parkii, Glycerin, Squalane, Ceramide 3. In die Basiscreme ist folgender weiterer Bestandteil eingearbeitet worden:

Silikonöl mit einem Silber-Gehalt von 0,65% w/w; das Silber liegt darin in Form von Partikeln mit einem mittleren Durchmesser von 10 nm vor; das Silber wird im Folgenden als "nanodisperses Silber" bezeichnet;
oder
in Pulverform vorliegende Agglomerate metallischen Silbers mit einer mittleren Porosität von 80% und einem mittleren Durchmesser von 5 µm; das Silber wird im Folgenden als "Agglomerat-Silber" bezeichnet.

Es wurden Cremes mit 0,01% w/w nanodispersem Silber sowie mit 0,1% w/w und 0,5% w/w Agglomerat-Silber hergestellt. Darüber hinaus wurde eine Creme mit 0,05% w/w nanodispersem Silber hergestellt, wobei das nanodisperse Silber hier aus einer Legierung, bestehend aus 99,5% w/w Silber, 0,49% w/w Zink und 0,01% w/w Kupfer, bestand. Weiterhin wurde eine Creme mit 1,5% w/w Agglomerat-Silber hergestellt, wobei das Agglomerat-Silber hier aus einer Legierung, bestehend aus 99,5% w/w Silber, 0,49% w/w Zink und 0,01% w/w Kupfer, bestand.

Zur Herstellung der Cremes wurden die Substanzen jeweils in einem 50 ml Becherglas vermischt, in einem Wasserbad auf 75°C für 20 Minuten erwärmt und dann mittels eines Ultraturrax (Janke und Kunkel, Antrieb T25, Statordurchmesser 25 mm, Rotordurchmesser 17 mm) für 5 Minuten dispergiert. Anschließend wurde die Creme abgekühlt und nochmals durchmischt.

In Fig. 2 zeigt jedes Feld eine x-y-Grafik bei der auf der x-Achse die Zeit und auf der y-Achse die optische Dichte aufgetragen ist. Die in den Spalten 1 bis 8 der Fig. 2 dargestellten Versuchsergebnisse sind in parallelen, den Reihen A bis H entsprechenden Versuchsansätzen A bis H mit folgenden Cremes ermittelt worden:
- Spalte 1, Reihen A-H:: Creme ohne Silber-Zusätze
- Spalte 2, Reihen A-H:: Creme mit 0,1% w/w Agglomerat-Silber
- Spalte 3, Reihen A-H:: Creme mit 0,5% w/w Agglomerat-Silber
- Spalte 4, Reihen A-H:: Creme mit 1,5% w/w Agglomerat-Silber, bestehend aus 99,5% w/w Silber, 0,49% w/w Zink und 0,01% w/w Kupfer
- Spalte 5, Reihen A-H:: Creme mit 0,01% w/w nanodispersem Silber
- Spalte 6, Reihen A-H:: Creme mit 0,05% w/w nanodispersem Silber bestehend aus 99,5% w/w Silber, 0,49% w/w Zink und 0,01% w/w Kupfer
- Spalte 7, Reihe A:: Positivkontrolle
- Spalte 7, Reihe B:: Negativkontrolle
- Spalte 7, Reihe C:: Leerwert
- Spalte 8, Reihen A-H:: Sterilkontrollen

Bei der Positivkontrolle wurde ein metallisches Silber enthaltendes Polymer eingesetzt. Die Werte zeigen, dass die eingesetzten Bakterien gegenüber Silber sensitiv sind und davon abgetötet werden können. Bei der Negativkontrolle wurde das gleiche Polymer eingesetzt, dass jedoch kein Silber enthielt. Beim Leerwert handelt es sich um einen in einer leeren Vertiefung der Mikrotiterplatte gemessenen Wert, der bei der Auswertung von allen Meßwerten subtrahiert wurde. Beizen Sterilkontrollen wurde jeweils nur Medium ohne Zusatz von Staphylococcus epidermidis eingesetzt, um zu zeigen, dass das Bakterienwachstum nicht vom Medium herrührt.

Die Ergebnisse lassen sich wie folgt zusammenfassen:

| Probenbezeichnung | Onset-OD [h] brutto | Onset-OD[h] netto | Wirkung |
|---|---|---|---|
| 1A-H Creme ohne Silber-Zusätze | 5,2 | 0 | nicht antibakteriell |
| 2A-H Creme mit 0,1 % w/w Agglomerat-Silber | 18,4 | 13,2 | hoch antibakteriell |
| 3A-H Creme mit 0,5% w/w Agglomerat-Silber | 32,2 | 27,0 | hoch antibakteriell |
| 4A-H Creme mit 1,5% w/wAgglomerat-Silber, bestehend aus 99,5% w/w Silber, 0,49% w/w Zink und 0,01 % w/w Kupfer | 37,9 | 32,7 | hoch antibakteriell |
| 5A-H Creme mit 0,01% w/w nanodispersem Silber | 35,3 | 30,1 | hoch antibakteriell |
| 6A-H Creme mit 0,05% w/w nanodispersem Silber, bestehend aus 99,5% w/w Silber, 0,49% w/w Zink und 0,01% w/w Kupfer | Limit | >42,8 | bakterizid |
| 7A/B Positivkontrolle/Negativkontrolle | Limit / 9,2 | - | OK |
| 8A-H Sterilkontrollen | Limit | - | OK |
| 7 C Leerwert | | - | OK |

"Onset-OD [h] brutto" bezeichnet die in Stunden bemessene Zeit, bis es zu einem exponentiellen Anstieg der optischen Dichte (OD) um 0,2 kam. "Onset-OD [h] netto" ergibt sich aus "Onset-OD [h] brutto" durch jeweiligen Abzug des für die Creme ohne Silber-Zusätze ermittelten Werts "Onset-OD [h] brutto". Bei parallelen Versuchsansätzen ist jeweils der Mittelwert angegeben. "Antibakteriell" bezeichnet eine Wirkung, bei der das Wachstum der Bakterien verzögert wird, während "bakterizid" eine Wirkung bezeichnet, bei der die Bakterien zu 100% abgetötet werden, so dass kein Bakterienwachstum mehr beobachtet werden kann.

Die Versuchsergebnisse zeigen, dass Agglomerat-Silber wie nanodisperses Silber hoch antibakteriell wirkt. Nanodisperses Silber ist bei geringeren Silberkonzentrationen wirksam als Agglomerat-Silber. Mit Agglomerat-Silber lässt sich jedoch noch immer eine hoch antibakterielle Wirkung erzielen. Sowohl die Wirkung des Agglomerat-Silbers als auch die Wirkung des nanodispersen Silbers ist in den Cremes gesteigert, die neben Silber zusätzlich Zink und Kupfer enthalten.

## Patentansprüche

1. Körperpflegemittel zum Auftragen auf Haut und/oder Mukosa, wobei darin metallisches Silber enthaltende, aus Metall gebildete poröse Partikel mit einem mittleren Durchmesser zwischen 1 und 100 µm enthalten sind, wobei die Partikel eine mittlere innere Porosität von mindestens 65% aufweisen.

2. Körperpflegemittel nach Anspruch 1, wobei die Partikel eine mittlere innere Porosität zwischen 65 und 95%, bevorzugt zwischen 65 und 90%, insbesondere zwischen 70 und 85%, vorzugsweise zwischen 75 und 85%, oder bevorzugt zwischen 85 und 95%, insbesondere zwischen 90 und 95%, aufweisen.

3. Körperpflegemittel nach Anspruch 1 oder 2, wobei die Partikel als Agglomerate metallischer Primärpartikel vorliegen.

4. Körperpflegemittel nach Anspruch 3, wobei die Primärpartikel einen mittleren Durchmesser zwischen 10 und 200 nm, vorzugsweise zwischen 15 und 80 nm, aufweisen.

5. Körperpflegemittel nach Anspruch 3 oder 4, wobei der mittlere Abstand zwischen den jeweils äußersten Primärpartikeln an der Oberfläche der Agglomerate im Bereich von 20 bis 200 nm, vorzugsweise 100 bis 200 nm, liegt.

6. Körperpflegemittel nach einem der vorhergehenden Ansprüche, wobei die Partikel eine schwammartige Struktur aufweisen.

7. Körperpflegemittel nach einem der vorhergehenden Ansprüche, wobei die Partikel einen mittleren Außendurchmesser von 2 bis 20 µm, vorzugsweise 2 bis 5 µm, aufweisen.

8. Körperpflegemittel nach einem der vorhergehenden Ansprüche, wobei die Partikel eine spezifische Oberfläche zwischen 2 und 10 m²/g, insbesondere zwischen 3 und 6 m²/g, vorzugsweise zwischen 3,5 und 4,5 m²/g, aufweisen.

9. Körperpflegemittel nach einem der vorhergehenden Ansprüche, wobei die Partikel mindestens zu 99% w/w, vorzugsweise zu 99,9% w/w, aus metallischem Silber bestehen.

10. Körperpflegemittel nach einem der vorhergehenden Ansprüche, wobei die Partikel Verunreinigungen von weniger als 5 ppm an Kalium, Natrium oder Chlor aufweisen.

11. Körperpflegemittel nach einem der vorhergehenden Ansprüche, wobei die Partikel bis zu 0,5% w/w metallisches Zink und/oder bis zu 0,5% w/w metallisches Kupfer enthalten.

12. Körperpflegemittel nach einem der vorhergehenden Ansprüche, wobei die Partikel aus einer Silber-Zink-Legierung oder einer Silber-Zink-Kupfer-Legierung gebildet sind.

13. Körperpflegemittel nach einem der vorhergehenden Ansprüche, wobei das Körperpflegemittel neben den Partikeln keine Konservierungsstoffe enthält.

14. Körperpflegemittel nach einem der vorhergehenden Ansprüche, wobei darin die Partikel in einem Trägermaterial enthalten sind, welches aus einem Silikonöl, einem Mineralöl, Glyzerin oder einem Salbenbestandteil besteht.

15. Körperpflegemittel nach einem der vorhergehenden Ansprüche, wobei das Körperpflegemittel ein, insbesondere medizinisch wirksames, Präparat, wie eine Emulsion, eine Lotion, ein Gel, eine Creme, eine Salbe, eine Heilsalbe, ein Puder, ein Kosmetikum, eine Hautschutzcreme oder -salbe, ein Desinfektionsmittel, eine Suspension, eine Seife, ein synthetisches Tensid, ein Badezusatz, ein Peelingpräparat, ein Gesichtswasser, ein Zahnpflegemittel, eine Zahncreme, ein Mundwasser, ein Haarshampoo oder ein Sonnenschutzmittel ist.

16. Verwendung von metallisches Silber enthaltenden, aus Metall gebildeten porösen Partikeln mit einem mittleren Durchmesser zwischen 1 und 100 µm zur Herstellung eines auf Haut und/oder Mukosa aufzutragenden Medikaments zur Behandlung einer Entzündung und/oder einer Infektion bei einem Säugetier oder Menschen, wobei die mittlere innere Porosität der Partikel mindestens 65% beträgt.

17. Verwendung nach Anspruch 16, wobei die mittlere innere Porosität der Partikel zwischen 65 und 95%, bevorzugt zwischen 65 und 90%, insbesondere zwischen 70 und 85%, vorzugsweise zwischen 75 und 85%, oder bevorzugt zwischen 85 und 95%, insbesondere zwischen 90 und 95%, beträgt.

18. Verwendung nach Anspruch 16 oder 17, wobei die Partikel als Agglomerate metallischer Primärpartikel vorliegen.

19. Verwendung nach Anspruch 18, wobei die Primärpartikel einen mittleren Durchmesser zwischen 10 und 200 nm, vorzugsweise zwischen 16 und 80 nm, aufweisen.

20. Verwendung nach Anspruch 18 oder 19, wobei der mittlere Abstand zwischen den jeweils äußersten Primärpartikeln an der Oberfläche der Agglomerate im Bereich von 20 bis 200 nm, vorzugsweise 100 bis 200 nm, liegt.

21. Verwendung nach einem der Ansprüche 16 bis 20, wobei die Partikel eine schwammartige Struktur aufweisen.

22. Verwendung nach einem der Ansprüche 16 bis 21, wobei die Partikel einen mittleren Außendurchmesser von 2 bis 20 µm, vorzugsweise 2 bis 5 µm, aufweisen.

23. Verwendung nach einem der Ansprüche 16 bis 22, wobei die Partikel eine spezifische Oberfläche zwischen 2 und 10 m²/g, insbesondere zwischen 3 und 6 m²/g, vorzugsweise zwischen 3,5 und 4,5 m²/g, aufweisen.

24. Verwendung nach einem der Ansprüche 16 bis 23, wobei die Partikel mindestens zu 99% w/w, vorzugsweise zu 99,9% w/w, aus metallischem Silber bestehen.

25. Verwendung nach einem der Ansprüche 16 bis 24, wobei die Partikel Verunreinigungen von weniger als 5 ppm an Kalium, Natrium oder Chlor aufweisen.

26. Verwendung nach einem der Ansprüche 16 bis 25, wobei die Partikel bis zu 0,5% w/w metallisches Zink und/oder bis zu 0,5% w/w metallisches Kupfer enthalten.

27. Verwendung nach einem der Ansprüche 16 bis 26, wobei die Partikel aus einer Silber-Zink-Legierung oder einer Silber-Zink-Kupfer-Legierung gebildet sind.

28. Verwendung nach einem der Ansprüche 16 bis 27, wobei das Medikament neben den Partikeln keine Konservierungsstoffe enthält.

29. Verwendung nach einem der Ansprüche 16 bis 28, wobei die Behandlung eine topische Behandlung ist.

30. Verwendung nach einem der Ansprüche 16 bis 29, wobei das Medikament eine Salbe, eine Creme oder ein Gel ist.

31. Verwendung nach einem der Ansprüche 16 bis 30, wobei in dem Medikament die Partikel in einem Trägermaterial enthalten sind, welches aus einem Silikonöl, einem Mineralöl, Glyzerin oder einem Salbenbestandteil besteht.

## Claims

1. A body care product intended to be applied to the skin and/or mucosa, wherein said product contains porous particles formed of metal and containing metallic silver the mean diameter of which is between 1 and 100 µm, wherein said particles have a mean inner porosity of at least 65%.

2. The body care product according to claim 1, wherein the particles have a mean inner porosity of between 65 and 95%, by preference between 65 and 90%, in particular between 70 and 85%, preferably between 75 and 85%, or by preference between 85 and 95%, in particular between 90 and 95%.

3. The body care product according to claim 1 or 2, wherein the particles are present in the form of agglomerates of metallic primary particles.

4. The body care product according to claim 3, wherein the primary particles have a mean diameter of between 10 and 200 nm, preferably between 15 and 80 nm.

5. The body care product according to claim 3 or 4, wherein the mean distance between the in each case outermost primary particles on the surface of the agglomerates is in the range of 20 to 200 nm, preferably 100 to 200 nm.

6. The body care product according to any one of the preceding claims, wherein the particles have a sponge-like structure.

7. The body care product according to any one of the preceding claims, wherein the particles have a mean outer diameter of 2 to 20 µm, preferably 2 to 5 µm.

8. The body care product according to any one of the preceding claims, wherein the particles have a specific surface of between 2 and 10 m²/g, in particular between 3 and 6 m²/g, preferably between 3.5 and 4.5 m²/g.

9. The body care product according to any one of the preceding claims, wherein the particles consist of at least 99% w/w, preferably 99.9% w/w, metallic silver.

10. The body care product according to any one of the preceding claims, wherein the particles comprise impurities of less than 5 ppm of potassium, sodium or chlorine.

11. The body care product according to any one of the preceding claims, wherein the particles contain up to 0.5% w/w of metallic zinc and/or up to 0.5% w/w of metallic copper.

12. The body care product according to any one of the preceding claims, wherein the particles are formed of a silver-zinc alloy or a silver-zinc-copper alloy.

13. The body care product according to any one of the preceding claims, wherein said body care product does not contain preservatives in addition to the particles.

14. The body care product according to any one of the preceding claims, wherein the particles are contained in said product in a carrier material consisting of a silicone oil, a mineral oil, glycerine or an ointment ingredient.

15. The body care product according to any one of the preceding claims, wherein said body care product is a preparation, in particular a medically active one, such as an emulsion, a lotion, a gel, a cream, an ointment, a healing ointment, a powder, a cosmetic product, a skin protection cream or ointment, a disinfectant, a suspension, a soap, a synthetic surfactant, a bath additive, a peeling preparation, a facial tonic, a dentifrice, a toothpaste, a mouthwash, a hair shampoo or a sunscreen product.

16. The use of porous particles formed of metal and containing metallic silver the mean diameter of which is between 1 and 100 µm for producing a medicament intended to be applied to skin and/or mucosa for the treatment of an inflammation and/or an infection in a mammal or human, wherein the mean inner porosity of said particles is at least 65%.

17. The use according to claim 16, wherein the mean inner porosity of the particles is between 65 and 95%, by preference between 65 and 90%, in particular between 70 and 85%, preferably between 75 and 85%, or by preference between 85 and 95%, in particular between 90 and 95%.

18. The use according to claim 16 or 17, wherein the particles are present in the form of agglomerates of metallic primary particles.

19. The use according to claim 18, wherein the primary particles have a mean diameter of between 10 and 200 nm, preferably between 16 and 80 nm.

20. The use according to claim 18 or 19, wherein the mean distance between the in each case outermost primary particles on the surface of the agglomerates is in the range of 20 to 200 nm, preferably 100 to 200 nm.

21. The use according to any one of claims 16 to 20, wherein the particles have a sponge-like structure.

22. The use according to any one of claims 16 to 21, wherein the particles have a mean outer diameter of 2 to 20 µm, preferably 2 to 5 µm.

23. The use according to any one of claims 16 to 22, wherein the particles have a specific surface of between 2 and 10 m²/g, in particular between 3 and 6 m²/g, preferably between 3.5 and 4.5 m²/g.

24. The use according to any one of claims 16 to 23, wherein the particles consist of at least 99% w/w, preferably 99.9% w/w, metallic silver.

25. The use according to any one of claims 16 to 24, wherein the particles comprise impurities of less than 5 ppm of potassium, sodium or chlorine.

26. The use according to any one of claims 16 to 25, wherein the particles contain up to 0.5% w/w of metallic zinc and/or up to 0.5% w/w of metallic copper.

27. The use according to any one of claims 16 to 26, wherein the particles are formed of a silver-zinc alloy or a silver-zinc-copper alloy.

28. The use according to any one of claims 16 to 27, wherein the medicament does not contain preservatives in addition to the particles.

29. The use according to any one of claims 16 to 28, wherein the treatment is a topical treatment.

30. The use according to any one of claims 16 to 29, wherein the medicament is an ointment, a cream or a gel.

31. The use according to any one of claims 16 to 30, wherein the particles are contained in the medicament in a carrier material consisting of a silicone oil, a mineral oil, glycerine or an ointment ingredient.

## Revendications

1. Produit de soin pour le corps à appliquer sur la peau et/ou les muqueuses, ce produit contenant des particules poreuses formées de métal contenant de l'argent métallique d'un diamètre moyen entre 1 et 100 µm, les particules présentant une porosité interne moyenne d'au moins 65 %.

2. Produit de soin pour le corps selon la revendication 1, les particules présentant une porosité interne moyenne entre 65 et 95 %, de préférence entre 65 et 90 %, en particulier entre 70 et 85 %, de manière préférée entre 75 et 85 % ou de préférence entre 85 et 95 %, en particulier entre 90 et 95 %.

3. Produit de soin pour le corps selon la revendication 1 ou 2, les particules se présentant sous forme d'agglomérats de particules primaires métalliques.

4. Produit de soin pour le corps selon la revendication 3, les particules primaires présentant un diamètre moyen entre 10 et 200 nm, de manière préférée entre 15 et 80 nm.

5. Produit de soin pour le corps selon la revendication 3 ou 4, la distance moyenne entre les particules primaires situées respectivement le plus à l'extérieur au niveau de la surface des agglomérats se situant dans la plage allant de 20 à 200 nm, de manière préférée allant 100 à 200 nm.

6. Produit de soin pour le corps selon l'une quelconque des revendications précédentes, les particules présentant une structure semblable à une éponge.

7. Produit de soin pour le corps selon l'une quelconque des revendications précédentes, les particules présentant un diamètre extérieur moyen allant de 2 à 20 µm, de manière préférée de 2 à 5 µm.

8. Produit de soin pour le corps selon l'une quelconque des revendications précédentes, les particules présentant une surface spécifique entre 2 et 10 m²/g, en particulier entre 3 et 6 m²/g, de manière préférée entre 3,5 et 4,5 m²/g.

9. Produit de soin pour le corps selon l'une quelconque des revendications précédentes, les particules se composant d'au moins 99 % en poids, de manière préférée d'au moins 99,9% en poids, d'argent métallique.

10. Produit de soin pour le corps selon l'une quelconque des revendications précédentes, les particules présentant des impuretés représentant moins de 5 ppm de potassium, de sodium ou de chlore.

11. Produit de soin pour le corps selon l'une quelconque des revendications précédentes, les particules contenant jusqu'à 0,5 % en poids de zinc métallique et/ou jusqu'à 0,5 % en poids de cuivre métallique.

12. Produit de soin pour le corps selon l'une quelconque des revendications précédentes, les particules étant formées à partir d'un alliage d'argent-zinc ou d'un alliage d'argent-zinc-cuivre.

13. Produit de soin pour le corps selon l'une quelconque des revendications précédentes, le produit de soin pour le corps ne contenant, outre les particules, pas de conservateurs.

14. Produit de soin pour le corps selon l'une quelconque des revendications précédentes, les particules étant contenues dans une substance de support, qui est composée d'une huile siliconée, d'une huile minérale, de glycérine ou d'un composant de pommade.

15. Produit de soin pour le corps selon l'une quelconque des revendications précédentes, le produit de soin pour le corps étant une préparation active en particulier au niveau médical, comme une émulsion, une lotion, un gel, une crème, une pommade, une pommade de traitement, une poudre, un cosmétique, une crème ou pommade de protection de la peau, un agent désinfectant, une suspension, un savon, un agent tensioactif synthétique, un additif de bain, une préparation de gommage, une lotion tonique ou pour le visage, un produit de soins dentaires, un dentifrice, un bain de bouche, un shampooing pour les cheveux ou un agent de protection contre le soleil.

16. Utilisation de particules poreuses contenant de l'argent métallique et formées à partir de métal d'un diamètre moyen entre 1 et 100 µm pour la préparation d'un médicament à appliquer sur la peau et/ou les muqueuses pour le traitement d'une inflammation et/ou d'une infection chez un mammifère ou chez l'homme, la porosité moyenne interne des particules s'élevant à au moins 65 %.

17. Utilisation selon la revendication 16, la porosité moyenne interne des particules s'élevant à d'entre 65 et 95 %, de préférence entre 65 et 90 %, en particulier entre 70 et 85 %, de manière préférée entre 75 et 85 % ou de préférence entre 85 et 95 %, en particulier entre 90 et 95 %.

18. Utilisation selon la revendication 16 ou 17, les particules se présentant sous la forme d'agglomérats de particules primaires métalliques.

19. Utilisation selon la revendication 18, les particules primaires présentant un diamètre moyen entre 10 et 200 nm, de manière préférée entre 16 et 80 nm.

20. Utilisation selon la revendication 18 ou 19, la distance moyenne entre les particules primaires situées respectivement le plus à l'extérieur au niveau de la surface des agglomérats se situant dans la plage allant de 20 à 200 nm, de manière préférée allant de 100 à 200 nm.

21. Utilisation selon l'une quelconque des revendications 16 à 20, les particules présentant une structure semblable à une éponge.

22. Utilisation selon l'une quelconque des revendications 16 à 21, les particules présentant un diamètre extérieur moyen allant de 2 à 20 µm, de manière préférée de 2 à 5 µm.

23. Utilisation selon l'une quelconque des revendications 16 à 22, les particules présentant une surface spécifique entre 2 et 10 m²/g, en particulier entre 3 et 6 m²/g, de manière préférée entre 3,5 et 4,5 m²/g.

24. Utilisation selon l'une quelconque des revendications 16 à 23, les particules se composant d'au moins 99 % en poids, de manière préférée d'au moins 99,9% en poids, d'argent métallique.

25. Utilisation selon l'une quelconque des revendications 16 à 24, les particules présentant des impuretés représentant moins de 5 ppm en potassium, en sodium ou en chlore.

26. Utilisation selon l'une quelconque des revendications 16 à 25, les particules contenant jusqu'à 0,5 % en poids de zinc métallique et/ou jusqu'à 0,5 % en poids de cuivre métallique.

27. Utilisation selon l'une quelconque des revendications 16 à 26, les particules étant formées à partir d'un alliage d'argent-zinc ou d'un alliage d'argent-zinc-cuivre.

28. Utilisation selon l'une quelconque des revendications 16 à 27, le médicament ne contenant, outre les particules, pas de conservateurs.

29. Utilisation selon l'une quelconque des revendications 16 à 28, le traitement étant un traitement topique.

30. Utilisation selon l'une quelconque des revendications 16 à 29, le médicament étant une pommade, une crème ou un gel.

31. Utilisation selon l'une quelconque des revendications 16 à 30, les particules du médicament étant contenues dans une substance de support, laquelle se compose d'une huile siliconée, d'une huile minérale, de glycérine ou d'un composant de pommade.
